# EUROPÄISCHE PATENTSCHRIFT

(11) **EP 3 351 956 B1**
(45) Veröffentlichungstag und Bekanntmachung des Hinweises auf die Patenterteilung: **16.03.2022**
(21) Anmeldenummer: 17152234.5
(22) Anmeldetag: 19.01.2017
(51) Int. Cl.: G01R 33/56, A61B 5/055

(54) **VERFAHREN ZUR KLASSIFIKATION VON MITTELS EINER MAGNETRESONANZ- FINGERPRINTING METHODE VON EINEM UNTERSUCHUNGSOBJEKT ERFASSTEN MAGNETRESONANZ-MESSDATEN**
METHOD FOR CLASSIFICATION OF MAGNETIC RESONANCE MEASURING DATA RECORDED BY MEANS OF A MAGNETIC RESONANCE FINGERPRINTING METHOD OF AN OBJECT TO BE EXAMINED
PROCÉDÉ DE CLASSIFICATION DE DONNÉES DE MESURE DE RÉSONANCE MAGNÉTIQUE DÉTECTÉES D'UN OBJET D'ANALYSE AU MOYEN D'UN PROCÉDÉ DE PRISE D'EMPREINTE PAR RÉSONANCE MAGNÉTIQUE

(43) Veröffentlichungstag der Anmeldung: 25.07.2018
(73) Patentinhaber: Siemens Healthcare GmbH, 91052 Erlangen (DE)
(72) Erfinder: Feiweier, Thorsten, 91099 Poxdorf (DE); Huwer, Stefan, 91056 Erlangen (DE); Nittka, Mathias, 91083 Baiersdorf (DE)

(56) Entgegenhaltungen:
- US-A1- 2015 301 141
- CHAITRA BADVE ET AL.: "Quantitative differentiation of prostate cancer from normal peripheral zone using Magnetic Resonance Fingerprinting (MRF)", PROCEEDINGS OF THE INTERNATIONAL SOCIETY FOR MAGNETIC RESONANCE IN MEDICINE, ISMRM, 23RD ANNUAL MEETING AND EXHIBITION, TORONTO, ONTARIO, CANADA, 30 MAY - 5 JUNE 2015, Nr. 3848, 15. Mai 2015 (2015-05-15), XP040669524,
- Simone Coppo ET AL: "Overview of Magnetic Resonance Fingerprinting", , 1. Februar 2016 (2016-02-01), XP055380755, Gefunden im Internet: URL:http://clinical-mri.com/wp-content/upl oads/2016/04/Gulani_MRF_MAGNETOM_Flash_ISM RM_2016.pdf [gefunden am 2017-06-12]
- Andrés Larroza ET AL: "Texture Analysis in Magnetic Resonance Imaging: Review and Considerations for Future Applications" In: "Assessment of Cellular and Organ Function and Dysfunction using Direct and Derived MRI Methodologies", 26. Oktober 2016 (2016-10-26), InTech, XP055395719, ISBN: 978-953-51-2723-9 DOI: 10.5772/64641, * Abschnitt "1. Introduction" Abschnitt "2.1 MRI Acquisition" Abschnitt "2.4 Feature extraction" Abschnitt "2.6 Classification" *
- L. KJAER ET AL.: "Texture Analysis in Quantitative MR Imaging", ACTA RADIOLOGICA, vol. 36, no. 2, 1 January 1995 (1995-01-01), pages 127-135, XP055737646, GB ISSN: 0284-1851

## Beschreibung

Die Erfindung betrifft Verfahren zur Klassifikation von mittels einer Magnetresonanz-Fingerprinting Methode von einem Untersuchungsobjekt erfassten Magnetresonanz-Messdaten, eine Klassifikationseinheit, ein Magnetresonanzgerät und ein Computerprogrammprodukt.

In einem Magnetresonanzgerät, auch Magnetresonanztomographiesystem genannt, wird üblicherweise der zu untersuchende Körper eines Untersuchungsobjekts, beispielsweise eines Patienten, eines gesunden Probanden, eines Tiers oder eines Phantoms, mit Hilfe eines Hauptmagneten einem relativ hohen Hauptmagnetfeld, beispielsweise von 1,5 oder 3 oder 7 Tesla, ausgesetzt. Zusätzlich werden mit Hilfe einer Gradientenspuleneinheit Gradientenschaltungen ausgespielt. Über eine Hochfrequenzantenneneinheit werden dann mittels geeigneter Antenneneinrichtungen Hochfrequenz-Pulse, beispielsweise Anregungspulse, ausgesendet, was dazu führt, dass die Kernspins bestimmter, durch diese Hochfrequenz-Pulse resonant angeregter Atome um einen definierten Flipwinkel gegenüber den Magnetfeldlinien des Hauptmagnetfelds verkippt werden. Während der Relaxation der Kernspins werden durch deren Präzession Hochfrequenz-Signale, so genannte Magnetresonanz-Signale, abgestrahlt, die mittels geeigneter Hochfrequenzantennen empfangen und dann weiterverarbeitet werden. Aus den so akquirierten Rohdaten können schließlich die gewünschten Bilddaten rekonstruiert werden.

Für eine bestimmte Messung ist daher eine bestimmte Magnetresonanz-Sequenz, auch Pulssequenz genannt, auszusenden, welche aus einer Folge von Hochfrequenz-Pulsen, beispielsweise Anregungspulsen und Refokussierungspulsen, sowie passend dazu koordiniert auszusendenden Gradientenschaltungen in verschiedenen Gradientenachsen entlang verschiedener Raumrichtungen besteht. Zeitlich passend hierzu werden Auslesefenster gesetzt, welche die Zeiträume vorgeben, in denen die induzierten Magnetresonanz-Signale erfasst werden.

Die Interpretation der mittels des Magnetresonanzgeräts akquirierten Magnetresonanz-Messdaten, insbesondere die Klassifikation von Gewebeklassen bzw. Lokalisierung von Pathologien, sowie daraus abgeleitete Differentialdiagnosen, erfordert typischerweise Fachwissen und viel Erfahrung des befundenden Radiologen. Wünschenswert wäre eine Verfügbarkeit von Magnetresonanz-Messdaten in einer Form, die einen direkten Rückschluss auf einen Gewebetypus erlaubt.

In der Regel liegen Magnetresonanz-Messdaten in Form von Wichtungsbildern vor. Mit unterschiedlichen Messtechniken lassen sich beispielsweise T1-gewichtete, T2-gewichtete oder diffusionsgewichtete Magnetresonanz-Messdaten erzeugen. Bei dieser Form von Magnetresonanz-Messdaten weisen die Intensitätswerte eine nicht genau spezifizierte Korrelation mit der T1-Relaxationszeit, T2-Relaxationszeit oder Diffusion auf. Insbesondere können die Intensitätswerte in Abhängigkeit von einem verwendeten Messprotokoll oder einem Typ des Magnetresonanzgeräts variieren. Eine direkte Ableitung von Gewebeklassen aus den Wichtungsbildern ist somit typischerweise nicht praktikabel.

Seit einiger Zeit sind quantitative Magnetresonanz-Verfahren bekannt, mit denen quantitative Gewebeparameterkarten aufgenommen werden können. So lassen sich beispielsweise aus mehreren Messungen mit unterschiedlichen Echozeiten (TE) quantitative T2-Relaxationskarten berechnen. Aus Messungen mit unterschiedlichen Flipwinkeln können T1-Relaxationskarten berechnet werden. Messungen mit unterschiedlichen b-Werten können ADC-Karten (apparent diffusion coefficient Karten) hervorbringen. In der Regel werden dabei separate Messungen für jede der zu ermittelnden Gewebeparameterkarten vorgenommen. Dies kann zu langen Messzeiten führen und das Risiko von räumlichen Fehlregistrierungen, beispielsweise bei Bewegung des Patienten, mit sich bringen.

Aus der Schrift Ma et al., "Magnetic Resonance Fingerprinting", Nature, 495, 187-192 (14 March 2013) ist eine mögliche Magnetresonanz-Fingerprinting Methode bekannt. Verschiedene Anwendung der Magnetresonanz-Fingerprinting Methode sind beispielsweise aus Patentanmeldungen bekannt. Beispielsweise eine Bestimmung einer ortsaufgelösten Verteilung einer Markierungssubstanz aus der US 2015/0366484 A1, eine Bewegungskorrektur von Magnetresonanz-Fingerprinting Messdaten aus der US 2016/0097830 A1, ein Verfahren zur Temperaturbestimmung aus der US 2016/0033604 A1, ein Verfahren zur Bestimmung von Auswirkungen einer Strahlentherapie aus der US 2016/0059041 A1 oder eine auf bestimmte Stoffe zugeschnittene Magnetresonanz-Fingerprinting Methode aus der US 2016/0061922 A1.

Die Magnetresonanz-Fingerprinting Methode stellt ein quantitatives Magnetresonanz-Verfahren dar, mittels welchem quantitative Werte von Gewebeparametern eines Untersuchungsobjekts und somit Gewebeparameterkarten bestimmt werden können. Ein Vorteil der Magnetresonanz-Fingerprinting Methode ist dabei, dass mehrere Gewebeparameter, beispielsweise eine T1-Relaxationszeit und eine T2-Relaxationszeit, gleichzeitig in einer einzelnen Messung akquiriert werden können. Derart kann die Magnetresonanz-Fingerprinting Methode einen Einsatz von mehreren verschiedenen Aufnahmesequenzen zum Erfassen der mehreren Gewebeparameter unnötig machen und so eine Komplexität und/oder eine Aufnahmezeit einer Magnetresonanz-Untersuchung verringern.

Vorliegende multiparametrische Magnetresonanz-Messdaten werden typischerweise pixelweise aufbereitet und interpretiert. Beispielsweise kann für jeden Gewebeparameter eine individuelle Gewebeparameterkarte, beispielsweise T1-Relaxationskarte, T2-Relaxationskarte und ADC-Karte generiert werden. Die verschiedenen Gewebeparameterkarten können dann vom Radiologen nebeneinander oder in Form einer Bildüberlagerung ("Fusion") visuell interpretiert werden. Zwar erleichtert das Vorliegen der Gewebeparameterkarten an Stelle von Wichtungsbildern die Identifizierung von Gewebeklassen, trotzdem erfordert die Interpretation der Gewebeparameterwerte pro Pixel viel Erfahrung. Insbesondere zeigt sich, dass typischerweise jeder einzelne Gewebeparameter für sich genommen nicht ausreicht, um eine robuste Gewebeklassifikation vornehmen zu können.

Alternativ können die multiparametrischen MagnetresonanzMessdaten voxelweise miteinander korreliert und entsprechend aufbereitet werden. Auf diese Weise können beispielsweise Voxel klassifiziert werden, die sowohl in einem vorgegebenen T1-Intervall als auch in einem vorgegebenen T2-Intervall liegen. Entsprechende Korrelationskarten lassen sich voxelweise berechnen und darstellen. Allerdings stellt sich heraus, dass auch die Voxel-Korrelationskarten unzureichend sind, um die Magnetresonanz-Messdaten robust in Gewebeklassen klassifizieren zu können.

Quantitative differentiation of prostate cancer from normal peripheral zone using Magnetic Resonance Fingerprinting (MRF), Badve et al., Proc. Intl. Soc. Mag. Reson. Med. 23 (2015), beschreibt ein Verfahren zur Nutzung von MRF zur Evaluierung von Prostatakrebs.

Overview of Magnetic Resonance Fingerprinting, Coppo et al., MAGNETOM_Flash (65) 2/2016, beschreibt die Grundlagen von MRF.

Texture Analysis in Magnetic Resonance lmaging: Review and Considerations for Future Applications, Larroza et al., Assessment of Cellular and Organ Function and Dysfunction using Direct and Derived MRI Methodologies, DOI: 10.5772/64641, beschreibt eine Übersicht über Verfahren zur Texturanalyse mittels MR-Bildgebung.

US 2015/301141 A1 beschreibt ein Verfahren zur Segmentierung und Klassifizierung von Gewebe mittels MRF. L. KJAER ET AL.: "Texture Analysis in Quantitative MR lmaging", Acta Radiologica, 36(2), beschreibt ein Verfahren zur Gewebecharakterisierung im Kopf mittels quantitativer MR-Bildgebung.

Der Erfindung liegt die Aufgabe zu Grunde, eine verbesserte Klassifikation von mittels einer Magnetresonanz-Fingerprinting Methode von einem Untersuchungsobjekt erfassten Magnetresonanz-Messdaten zu ermöglichen. Die Aufgabe wird durch die Merkmale der unabhängigen Ansprüche gelöst. Vorteilhafte Ausgestaltungen sind in den Unteransprüchen beschrieben.

Die erfindungsgemäßen Verfahren zur Klassifikation von mittels einer Magnetresonanz-Fingerprinting Methode von einem Untersuchungsobjekt erfassten Magnetresonanz-Messdaten umfassen folgende gemeinsame Verfahrensschritte:
- Erfassen von Magnetresonanz-Messdaten des Untersuchungsobjekts, wobei die Magnetresonanz-Messdaten mehrere mittels einer Magnetresonanz-Fingerprinting Methode erfasste Magnetresonanz-Signalverläufe von verschiedenen Voxels umfassen,
- Ableiten zumindest eines Texturparameters aus den Magnetresonanz-Messdaten,
- Klassifikation der Magnetresonanz-Messdaten in zumindest eine Gewebeklasse unter Verwendung des zumindest einen Texturparameters und
- Bereitstellen der klassifizierten Magnetresonanz-Messdaten.
wobei gemäß eines ersten erfindungsgemäßen Verfahrens:
- das Ableiten des zumindest einen Texturparameters eine Rekonstruktion von zumindest einer Texturparameterkarte umfasst, wobei ein für die Rekonstruktion der zumindest einen Texturparameterkarte verwendetes Magnetresonanz-Fingerprinting Modell einen räumlichen Zusammenhang der mehreren MagnetresonanzSignalverläufe berücksichtigt, und - die Klassifikation der Magnetresonanz-Messdaten unter Verwendung der zumindest einen Texturparameterkarte erfolgt
und gemäß eines zweiten erfindungsgemäßen Verfahrens der zumindest eine Texturparameter zeitaufgelöst über eine Zeitspanne des Erfassens der mehreren Magnetresonanz-Signalverläufe abgeleitet wird.

Das Erfassen der Magnetresonanz-Messdaten kann eine Akquisition der Magnetresonanz-Messdaten mittels eines Magnetresonanzgeräts oder ein Laden von bereits akquirierten Magnetresonanz-Messdaten aus einer Datenbank umfassen.

Das Erfassen der Magnetresonanz-Messdaten mittels der Magnetresonanz-Fingerprinting Methode sieht ein Erfassen der mehreren Magnetresonanz-Signalverläufe von verschiedenen Voxels aus einem Untersuchungsbereich des Untersuchungsobjekts vor. Die mehreren Magnetresonanz-Signalverläufe werden typischerweise anschließend mit einer Signalmodellierung abgeglichen. Die mehreren Magnetresonanz-Signalverläufe werden dabei typischerweise mittels eines pseudorandominisierten bzw. inkohärenten Aufnahmeschemas akquiriert. Alternativ sind auch regelmäßige bzw. kohärente und/oder vollständig abgetastete Aufnahmeschemata zur Akquisition der mehreren Magnetresonanz-Signalverläufe denkbar. Grundsätzlich ist hierbei eine Messung der mehreren MagnetresonanzSignalverläufe in einem Nicht-Gleichgewichtszustand sinnvoll. Ein erfasster Magnetresonanz-Signalverlauf der mehreren Magnetresonanz-Signalverläufe gibt insbesondere an, wie sich ein Signalwert eines in dem jeweiligen Voxel aufgenommenen Magnetresonanz-Signals während des Erfassens des Magnetresonanz-Signalverlaufs verändert. Der Voxel gibt dabei den Bereich an, aus welchem der Magnetresonanz-Signalverlauf erfasst wird.

In einem typischen Anwendungsfall werden die mehreren Magnetresonanz-Signalverläufe simultan aus mehreren Voxeln erfasst. Dafür können beispielsweise mehrere Rohbilder mittels der Magnetresonanz-Fingerprinting Methode in einer zeitlichen Abfolge akquiriert werden, wobei dann die mehreren Magnetresonanz-Signalverläufe über die einzelnen Voxel der mehreren Rohbilder gebildet werden können. Eine Zeitauflösung der Magnetresonanz-Signalverläufe wird dabei insbesondere durch einen zeitlichen Abstand der Aufnahme von verschiedenen Magnetresonanz-Signalen bzw. der mehreren Rohbilder gebildet.

Die Magnetresonanz-Fingerprinting Methode umfasst insbesondere, dass für die Aufnahme der verschiedenen Magnetresonanz-Signale verschiedene Aufnahmeparameter gesetzt werden. Die Aufnahmeparameter können dabei über den Zeitraum der Akquisition der Magnetresonanz-Signalverläufe in einer pseudorandominisierten bzw. inkohärenten Weise variiert werden. Mögliche Aufnahmeparameter, welche bei der Akquisition der Magnetresonanz-Signalverläufe verändert werden, sind beispielsweise eine Echozeit, eine Repetitionszeit, eine Ausbildung und/oder Anzahl von Hochfrequenz-Pulsen, eine Ausbildung und/oder Anzahl von Gradientenpulsen, eine Diffusionskodierung usw. Es ist auch denkbar, dass der Nicht-Gleichgewichtszustand bei der Akquisition der mehreren Signalverläufe durch geeignete Präparationsmodule, insbesondere umfassend einen Sättigungspuls und/oder einen Inversionspuls, generiert wird. Derart kann mittels der Magnetresonanz-Fingerprinting Methode ein für den Voxel charakteristischer Magnetresonanz-Signalverlauf, ein sogenannter Fingerabdruck (Fingerprint) des Voxels, erfasst werden.

Der zumindest eine Texturparameter wird insbesondere mittels eines Ableitungsalgorithmus aus den Magnetresonanz-Messdaten abgeleitet. Die Magnetresonanz-Messdaten gehen also insbesondere als Eingangsdaten in den Ableitungsalgorithmus ein, welcher als Ausgangsdaten den zumindest einen Texturparameter aufweist. Dabei kann der zumindest eine Texturparameter direkt aus den Magnetresonanz-Messdaten, insbesondere direkt aus den mehreren Magnetresonanz-Signalverläufen, abgeleitet werden. Es ist auch denkbar, dass die Magnetresonanz-Messdaten nur indirekt in den Ableitungsalgorithmus eingehen, d.h. dass insbesondere zunächst die Magnetresonanz-Signalverläufe weiterverarbeitet werden und anschließend das Produkt der Weiterverarbeitung der Magnetresonanz-Signalverläufe in den Ableitungsalgorithmus eingeht. Verschiedene Möglichkeiten, wie die Magnetresonanz-Messdaten in den Ableitungsalgorithmus eingehen können, sind in folgenden Absätzen und Ausführungsformen beschrieben.

Unter einer Textur lässt sich typischerweise eine bestimmte Struktur, insbesondere eine Gewebestruktur, eines Bildinhalts bzw. Messdateninhalts beschreiben. Derart kann der zumindest eine Texturparameter die bestimmte Struktur, insbesondere die Gewebestruktur, charakterisieren. Der zumindest eine Texturparameter kann derart zur Quantifizierung von räumlichen Merkmalen in den Magnetresonanz-Messdaten dienen. So kann der zumindest eine Texturparameter beispielsweise Informationen über eine räumliche Anordnung von gemessenen Intensitäten in den Magnetresonanz-Messdaten umfassen. Alternativ oder zusätzlich kann der zumindest eine Texturparameter auch zeitliche Muster in den Magnetresonanz-Messdaten charakterisieren, beispielsweise wie sich die Magnetresonanz-Signale in den mehreren Magnetresonanz-Signalverläufen zeitlich entwickeln.

Es können ein oder mehrere Texturparameter aus den Magnetresonanz-Messdaten abgeleitet werden. Verschiedene Möglichkeiten zum Ableiten von Texturparametern sind dabei dem Fachmann aus dem Umfeld der Bildverarbeitung bekannt. Beispielsweise kann der zumindest eine Texturparameter mittels eines statistischen Verfahrens gewonnen werden und somit einen statistischen Texturparameter umfassen. Beispielsweise für solche statistischen Texturparameter sind: Mittelwert, Varianz, Schiefe, Markov Random Fields, Co-Occurence Matrizen. Es ist auch möglich, dass der zumindest eine Texturparameter einen strukturellen Texturparameter umfasst. Dabei kann der strukturelle Texturparameter beispielsweise eine Energieverteilung, eine Entropie, einen Kontrast, eine Homogenität in den Magnetresonanz-Messdaten charakterisieren. Selbstverständlich ist auch ein Ableiten von weiteren, dem Fachmann als sinnvoll erscheinenden Texturparametern denkbar.

Die Klassifikation der Magnetresonanz-Messdaten erfolgt insbesondere mittels eines Klassifikationsalgorithmus, welcher als Eingangsdaten den zumindest einen Texturparameter und als Ausgangsdaten die klassifizierten Magnetresonanz-Messdaten aufweist. Die Magnetresonanz-Messdaten selbst können zusätzlich zum zumindest einen Texturparameter als Eingangsdaten in den Klassifikationsalgorithmus eingehen, um beispielsweise eine ungefähre Orientierung bei der Klassifikation in die zumindest eine Gewebeklasse zu bilden. Typischerweise wird jedoch der zumindest eine Texturparameter anstelle der Magnetresonanz-Messdaten für die Klassifikation der Magnetresonanz-Messdaten verwendet. Der zumindest eine Texturparameter kann somit eine neue Repräsentation der Inhalte der Magnetresonanz-Messdaten darstellen.

Die Magnetresonanz-Messdaten können in eine oder mehrere Gewebeklassen klassifiziert werden. Dass die MagnetresonanzMessdaten klassifiziert werden kann insbesondere umfassen, dass direkt die Magnetresonanz-Messdaten, also die mehreren Magnetresonanz-Signalverläufe klassifiziert werden, oder dass aus den Magnetresonanz-Messdaten rekonstruierte Magnetresonanz-Bilddaten klassifiziert werden. Nach der Klassifikation ist zu verschiedenen Teilen der Magnetresonanz-Messdaten bzw. der aus den Magnetresonanz-Messdaten rekonstruierten Magnetresonanz-Bilddaten insbesondere die passende zumindest eine Gewebeklasse zugeordnet. Die klassifizierten Magnetresonanz-Messdaten können demnach die Information umfassen, von welcher Gewebeklasse des Untersuchungsobjekts bestimmte Teile der Magnetresonanz-Messdaten bzw. der aus den MagnetresonanzMessdaten rekonstruierten Magnetresonanz-Bilddaten erfasst worden sind.

Die zumindest eine Gewebeklasse kann einen Gewebetyp bzw. eine Gewebesorte charakterisieren, von dem die MagnetresonanzMessdaten erfasst worden sind. So kann die zumindest eine Gewebeklasse beispielsweise Fettgewebe, Weichteilgewebe, Knochengewebe, Knorpelgewebe, Muskelgewebe, usw. umfassen. Auch kann die zumindest eine Gewebeklasse Gewebe von einem bestimmten Organtyp, beispielsweise Lebergewebe, Lungengewebe, weiße Hirnsubstanz, graue Hirnsubstanz, usw. umfassen. Die zumindest eine Gewebeklasse kann zusätzlich auch Luft bzw. Hintergrund umfassen. Es ist auch denkbar, dass die zumindest eine Gewebeklasse einen Fremdkörper im Körper des Untersuchungsobjekts, beispielsweise ein Implantatmaterial, umfasst. In einem besonders wichtigen Anwendungsfall kann die zumindest eine Gewebeklasse eine Gewebetypisierung in dem Sinne, ob es sich um normales bzw. physiologisches Gewebe oder um auffälliges bzw. pathologisches Gewebe handelt, umfassen. So kann beispielsweise eine Klassifikation in normales Gewebe und Tumorgewebe durchgeführt werden. Selbstverständlich kann auch eine Klassifikation in andere pathologische Gewebesorten, wie beispielsweise Narbengewebe usw., erfolgen. Selbstverständlich sind weitere, dem Fachmann als sinnvoll erscheinende Gewebeklassen denkbar, in welche die MagnetresonanzMessdaten klassifiziert werden können.

Für die Klassifikation der Magnetresonanz-Messdaten, insbesondere der beim Ableiten des zumindest einen Texturparameters gewonnenen Merkmalsvektoren, können verschiedene Verfahren eingesetzt werden. Beispielsweise ist ein Einsatz von einem k-Nearest-Neighbour Verfahren, einer statistischen Bayes-Klassifikation, von Support Vector Machines, usw. möglich. Selbstverständlich sind weitere, dem Fachmann als sinnvoll erscheinende Klassifikationsverfahren denkbar. In der Regel ist die zu erzielende Güte der Klassifikatoren mit der Eignung des zumindest einen Texturparameters bezüglich der Separierbarkeit verschiedener Gewebeklassen korreliert.

Neben den genannten Verfahren ist auch der Einsatz eines selbst lernenden Verfahrens für die Klassifikation der Magnetresonanz-Messdaten denkbar. Bei einem solchen Verfahren wird üblicherweise direkt die Abbildung von einem Teil der Magnetresonanz-Messdaten zu einer Gewebeklasse über eine hinreichend große Traningsstichprobe gelernt. Ein möglicher geeigneter Vertreter dieser Klasse an Klassifikatoren sind die Deep Convolutional Neural Networks. Hier wird insbesondere der abzuleitende Texturparameter nicht mehr explizit beschrieben, sondern im Verfahren der automatischen Klassifikation eines Teils der Magnetresonanz-Messdaten implizit mitgelernt.

Das Bereitstellen der klassifizierten MagnetresonanzMessdaten kann eine Anzeige der klassifizierten Magnetresonanz-Messdaten auf einer Anzeigeeinheit umfassen. Hierbei können beispielweise die Magnetresonanz-Messdaten mit ihrer zugehörigen Klassifikation, beispielsweise in Form einer Segmentierung und/oder farblichen Überlagerung, angezeigt werden. Das Bereitstellen der klassifizierten MagnetresonanzMessdaten kann auch ein Abspeichern der klassifizierten Magnetresonanz-Messdaten in einer Datenbank und/oder eine Weitergabe der klassifizierten Magnetresonanz-Messdaten an eine Weiterverarbeitungseinheit zur Weiterverarbeitung umfassen. Dass klassifizierte Magnetresonanz-Messdaten bereitgestellt, also beispielsweise angezeigt und/oder abgespeichert, werden, kann insbesondere umfassen, dass aus den MagnetresonanzMessdaten rekonstruierte Magnetresonanz-Bilddaten in klassifizierter Form bereitgestellt werden.

Dem vorgeschlagenen Vorgehen liegt die Überlegung zugrunde, dass eine einfache voxelweise Zuordnung bzw. Klassifikation von Gewebeklassen in rekonstruierten Magnetresonanz-Bilddaten häufig schwierig ist, da das zu klassifizierende Gewebe typischerweise räumlich strukturiert ist. So kann sich beispielsweise ein pathologisches Gewebe von gesundem Gewebe dadurch unterscheiden, dass sich Magnetresonanz-Messdaten bzw. aus Magnetresonanz-Messdaten rekonstruierte Magnetresonanz-Bilddaten räumlich in charakteristischer Weise verändern. Eine solche charakteristische Variation kann beispielsweise eine prägnante lokale Schwankung ("gesprenkelt") oder eine schleichende Veränderung ("diffus") umfassen. Das geschulte Auge des Radiologen kann solche Strukturen erkennen - eine Pixel-basierte Klassifikation ist allerdings nicht praktikabel.

Gemäß dem vorgeschlagenen Vorgehen soll zumindest ein Texturparameter zur verbesserten Klassifikation von mittels einer Magnetresonanz-Fingerprinting Methode erfassten Magnetresonanz-Messdaten eingesetzt werden. Derart können räumliche und/oder zeitliche Merkmale bei der Klassifikation der Magnetresonanz-Messdaten eingesetzt werden. Derart kann die Magnetresonanz-Fingerprinting Methode derart sinnvoll erweitert werden, dass insbesondere nicht mehr nur die MagnetresonanzSignalverläufe der einzelnen Voxel getrennt voneinander betrachtet werden. Vielmehr kann die Klassifikation Magnetresonanz-Messdaten der Magnetresonanz-Fingerprinting Methode mittels einer Betrachtung von räumlichen Nachbarschaftsbeziehungen der Magnetresonanz-Signalverläufe verbessert werden.

Die Magnetresonanz-Fingerprinting Methode eignet sich dabei besonders für die Klassifikation der MagnetresonanzMessdaten, da mittels der Magnetresonanz-Fingerprinting Methode inhärent multiparametrische quantitative Gewebeparameter ermittelt werden können. Da so die Magnetresonanz-Fingerprinting Methode über verschiedene Messungen hinweg absolute bzw. vergleichbare Messergebnisse liefern kann, sind die mittels der Magnetresonanz-Fingerprinting Methode gewonnenen Magnetresonanz-Messdaten besonders geeignet für die Klassifikation in die zumindest eine Gewebeklasse.

Gerade der Einsatz des zumindest einen Texturparameters kann die Klassifikation der mittels der Magnetresonanz-Fingerprinting Methode erhaltenen Magnetresonanz-Messdaten vorteilhaft weiterentwickeln, da die Berücksichtigung räumlicher und/oder zeitlicher Strukturen in den MagnetresonanzMessdaten eine robustere Differenzierung von Gewebeklassen ermöglichen kann. Derart können im Vergleich zu einer herkömmlichen rein voxel-basierten Auswertung der mittels der Magnetresonanz-Fingerprinting Methode erhaltenen Magnetresonanz-Messdaten Normabweichungen effizienter lokalisiert werden. Üblicherweise ist nämlich die Zusammensetzung von Gewebe, zum Beispiel in einem Tumor, zu komplex, als dass sich aus den in einem einzelnen Voxel gemessenen Gewebeparametern, wie beispielsweise der T1-Relaxationszeit und T2-Relaxationszeit, zuverlässig ein bestimmter Gewebetyp oder gar eine Pathologie zuordnen ließe. Aus diesem Grund kann die Kombination der quantitativen Magnetresonanz-Fingerprinting Methode mit der Betrachtung der räumlichen und/oder zeitlichen Merkmale einen erheblichen Vorteil für die Klassifikation der Magnetresonanz-Messdaten darstellen.

Gemäß des ersten oben definierten erfindungsgemäßen Verfahrens wird der zumindest eine Texturparameter unmittelbar aus den mehreren Magnetresonanz-Signalverläufen abgeleitet. Dafür werden insbesondere die mehreren Magnetresonanz-Signalverläufe kombiniert zeitlich und räumlich analysiert, um den zumindest einen Texturparameter zu extrahieren. Die bei der herkömmlichen Magnetresonanz-Fingerprinting Rekonstruktion übliche Ermittlung der mehreren Gewebeparameterkarten kann somit entfallen.

Für das direkte Ableiten des zumindest einen Texturparameters aus den mehreren Magnetresonanz-Signalverläufen erfolgt vorteilhafterweise nicht nur lediglich der übliche voxelbasierte Vergleich der Magnetresonanz-Signalverläufe mit den Datenbank-Signalverläufen. Vielmehr wird die Magnetresonanz-Fingerprinting Rekonstruktion vorteilhafterweise um die Berücksichtigung einer räumlichen Komponente, die charakteristische Texturmerkmale enthalt, erweitert.

Gemäß dieser erfindungsgemäßen Ausführungsform berücksichtigt also das für die Rekonstruktion der zumindest einen Texturparameterkarte verwendete Magnetresonanz-Fingerprinting Modell einen räumlichen Zusammenhang der mehreren MagnetresonanzSignalverläufe. So kann bereits bei der Modellierung der zeitlichen Signalevolution der mehreren MagnetresonanzSignalverläufe eine lokale räumliche Strukturierung des Gewebes des Untersuchungsobjekts berücksichtigt werden. Eine höhere Komplexität dieses Magnetresonanz-Fingerprinting Modells kann durch geeignete Kompressionsverfahren, beispielsweise einer PCA (Principal Component Analysis), verringert werden. Durch die Berücksichtigung des räumlichen Zusammenhangs der mehreren Magnetresonanz-Signalverläufe kann direkt die zumindest eine Texturparameterkarte aus den Magnetresonanz-Fingerprinting Signalverläufen abgeleitet werden. So kann besonders einfach die Klassifikation der MagnetresonanzMessdaten auf Basis der zumindest einen Texturparameterkarte, insbesondere auf Basis einer Kombination aus verschiedenen Texturparametern, durchgeführt werden.

Ein zweites erfindungsgemäßes Verfahren zur Klassifikation von mittels einer Magnetresonanz-Fingerprinting Methode von einem Untersuchungsobjekt erfassten Magnetresonanz-Messdaten, umfasst folgende Verfahrensschritte:
- Erfassen von Magnetresonanz-Messdaten des Untersuchungsobjekts, wobei die Magnetresonanz-Messdaten mehrere mittels einer Magnetresonanz-Fingerprinting Methode erfasste Magnetresonanz-Signalverläufe von verschiedenen Voxels umfassen,
- Ableiten zumindest eines Texturparameters aus den Magnetresonanz-Messdaten,
- Klassifikation der Magnetresonanz-Messdaten in zumindest eine Gewebeklasse unter Verwendung des zumindest einen Texturparameters und
- Bereitstellen der klassifizierten Magnetresonanz-Messdaten, wobei
   der zumindest eine Texturparameter zeitaufgelöst über eine Zeitspanne des Erfassens der mehreren Magnetresonanz-Signalverläufe abgeleitet wird.

Derart wird insbesondere nicht nur zumindest ein statistischer Texturparameter über die während der gesamten Zeitspanne erfassten Magnetresonanz-Messdaten bestimmt. Vielmehr variiert der zumindest eine Texturparameter insbesondere über die Zeitspanne des Erfassens der mehreren MagnetresonanzSignalverläufe. Dabei ist die Zeitspanne des Erfassens der mehreren Magnetresonanz-Signalverläufe insbesondere durch den Startzeitpunkt und Endzeitpunkt des Erfassens der mehreren Magnetresonanzsignalverläufe definiert. Der zumindest eine Texturparameter kann auch nur über einen Teil der Zeitspanne des Erfassens der mehreren Magnetresonanz-Signalverläufe variieren.

In einem bevorzugten Anwendungsfall umfasst dabei das Ableiten des zumindest einen zeitaufgelösten Texturparameters eine Rekonstruktion von mehreren zeitaufgelösten Texturparameterkarten über unterschiedliche zeitliche Abschnitte der Zeitspanne des Erfassens der mehreren Magnetresonanz-Signalverläufe hinweg. Die Klassifikation der MagnetresonanzMessdaten kann dann unter Verwendung der mehreren zeitaufgelösten Texturparameterkarten erfolgen. Dafür kann für jedes mittels der Magnetresonanz-Fingerprinting Methode erfasste Rohbild jeweils eine Texturparameterkarte bestimmt werden. Alternativ kann auch jeweils für eine Sequenz von mehreren aufeinanderfolgend erfassten Rohbildern eine Texturparameterkarte bestimmt werden. Als Zwischenresultat liegt somit insbesondere eine zeitliche Abfolge unterschiedlicher Texturparameterkarten vor. Diese können dann in die Klassifikation der Magnetresonanz-Messdaten eingehen, wobei die zeitliche Komponente besonders vorteilhafte Zusatzinformationen für die Klassifikation bieten kann.

Eine Ausführungsform sieht vor, dass die Klassifikation der Magnetresonanz-Messdaten aus dem zumindest einem zeitaufgelöstem Texturparameter unter Verwendung einer Datenbank erfolgt, wobei die Datenbank Informationen über eine zeitliche Evolution des zumindest einen Texturparameters über die Zeitspanne des Erfassens der mehreren Magnetresonanz-Signalverläufe in Abhängigkeit von der zumindest einen Gewebeklasse umfasst.

Anstelle der herkömmlichen Magnetresonanz-Fingerprinting Rekonstruktion, bei der die Magnetresonanz-Signalverläufe mit Datenbank-Signalverläufen, insbesondere über Amplitude und Phase, verglichen werden, erfolgt insbesondere eine Magnetresonanz-Fingerprinting Rekonstruktion unter Verwendung des zumindest einem zeitaufgelöstem Texturparameters. Dafür wird insbesondere der zeitaufgelöste Texturparameter mit Datenbankverläufen des zeitaufgelösten Texturparameters, welche die zeitliche Evolution des zumindest einen Texturparameters über die Zeitspanne des Erfassens der mehreren Magnetresonanz-Signalverläufe in Abhängigkeit von der zumindest einen Gewebeklasse beschreiben, verglichen. In der Datenbank sind hierbei insbesondere mehrere Datenbankverläufe hinterlegt, welche die zeitliche Evolution des zumindest einen Texturparameters für mehrere unterschiedliche Gewebeklassen beschreiben. Die Datenbankverläufe können dabei für unterschiedliche Gewebetypen, aber auch auf Grundlage von Gewebemerkmalen bzw. Gewebeeigenschaften (z.B. "normale" und "auffällige" Textur-Evolutionen) bestimmt werden.

Zu den mehreren Datenbankverläufen sind also insbesondere die unterschiedlichen Gewebeklassen, in welche die Magnetresonanz-Messdaten klassifiziert werden sollen, hinterlegt. Für die Klassifikation kann derjenige Datenbankverlauf ermittelt werden, welcher am besten mit der gemessenen zeitlichen Evolution des zumindest einen Texturparameters übereinstimmt. Hierfür kann beispielsweise ein Maximum eines inneren Produkts aus beiden Vektoren bestimmt werden. Als Klassifikationsergebnis kann dann diejenige Gewebeklasse gewählt werden, welcher dem am besten übereinstimmenden Datenbankverlauf zugeordnet ist. Derart kann als Ergebnis der Rekonstruktion der Magnetresonanz-Fingerprinting Messdaten vorteilhafterweise direkt die gewünschte Klassifikation vorliegen.

Eine Ausführungsform sieht vor, dass die Klassifikation der Magnetresonanz-Messdaten aus dem zumindest einem zeitaufgelöstem Texturparameter unter Verwendung zumindest einer Filteroperation erfolgt.

Als Alternative zu der Suche nach der besten Übereinstimmung der gemessenen zeitlichen Evolution des zumindest einen Texturparameters mit den Datenbankverläufen kann eine Modell- bzw. Filter-basierte Analyse der gemessenen zeitlichen Evolution des zumindest einen Texturparameters erfolgen. Hierbei kann zumindest eine dedizierte Filteroperation zum Extrahieren von Gewebecharakteristika für die Klassifikation in die Gewebeklassen aus der gemessenen zeitlichen Evolution des zumindest einen Texturparameters eingesetzt werden. Geeignet ist dabei beispielsweise eine Kalman-Filteroperation. Auch können nicht-lineare Zustandsmodelle, beispielsweise mit erweiterten Kalman-Filteroperationen oder mit sequenziellen Monte-Carlo-Verfahren, modelliert werden. Eine weitere Möglichkeit für die Filteroperation ist die Verwendung eines selbst-lernenden Filters, beispielsweise auf Basis eines neuronalen Netzwerks (auch als "deep learning" bezeichnet). In diesem Fall ist ein Training mit Trainings-Datensätzen sinnvoll. In diesem Fall kann vorteilhafterweise auf das Erstellen der dedizierten Filteroperation bzw. des dedizierten Modells verzichtet werden.

Eine Ausführungsform sieht vor, dass zumindest eine Längenskala im Ortsraum, auf deren Basis der zumindest eine Texturparameter abgeleitet wird, basierend auf zumindest einem der folgenden Elemente bestimmt wird: eine anatomische Region, von der die Magnetresonanz-Messdaten erfasst worden sind, eine klinische Fragestellung für das Erfassen der Magnetresonanz-Messdaten, eine bei dem Untersuchungsobjekt zu erwartende Pathologie, zuvor von dem Untersuchungsobjekt erfasste medizinische Bilddaten.

Wenn ein räumlicher Texturparameter aus den MagnetresonanzMessdaten ableitet werden soll, macht es Sinn die Längenskala im Ortsraum, auf deren Basis der zumindest eine Texturparameter abgeleitet wird, geeignet zu definieren. Derart kann der zumindest eine Texturparameter besonders geeignet für die Klassifikation der Magnetresonanz-Messdaten abgeleitet werden. Informationen über das Untersuchungsobjekt bzw. über die klinische Fragestellung können dabei eine geeignete Grundlage für die Definition der Längenskala sein. Ebenfalls ist es denkbar, dass Informationen aus einem anatomischen Atlas zur Bestimmung der Längenskala hinzugezogen werden.

Die Längenskala kann für die gesamte Magnetresonanz-Untersuchung konstant sein. Die Längenskala kann auch abhängig von der Position, von der die Magnetresonanz-Messdaten erfasst werden, variieren. Alternativ oder zusätzlich können in einer hierarchischen Analyse sukzessiv unterschiedliche Längenskalen (z.B. von grob nach fein) für das Ableiten des zumindest einen Texturparameters verwendet werden, wodurch weitere Informationen für eine robuste Klassifikation in die zumindest eine Gewebeklasse gewonnen werden können.

Die erfindungsgemäße Klassifikationseinheit umfasst eine Recheneinheit, wobei die Klassifikationseinheit zum Ausführen eines erfindungsgemäßen Verfahrens ausgebildet ist.

Derart ist die erfindungsgemäße Klassifikationseinheit zum Ausführen eines Verfahrens zur Klassifikation von mittels einer Magnetresonanz-Fingerprinting Methode von einem Untersuchungsobjekt erfassten Magnetresonanz-Messdaten ausgebildet. Dafür umfasst die erfindungsgemäße Klassifikationseinheit insbesondere eine Messdatenerfassungseinheit zum Erfassen von Magnetresonanz-Messdaten des Untersuchungsobjekts, wobei die Magnetresonanz-Messdaten mehrere mittels einer Magnetresonanz-Fingerprinting Methode erfasste Magnetresonanz-Signalverläufe umfassen. Die erfindungsgemäße Klassifikationseinheit umfasst weiterhin insbesondere eine Ableitungseinheit zum Ableiten zumindest eines Texturparameters aus den Magnetresonanz-Messdaten. Die erfindungsgemäße Klassifikationseinheit umfasst weiterhin insbesondere einen Klassifikator zur Klassifikation der Magnetresonanz-Messdaten in zumindest eine Gewebeklasse unter Verwendung des zumindest einen Texturparameters. Die erfindungsgemäße Klassifikationseinheit umfasst weiterhin insbesondere eine Bereitstellungseinheit zum Bereitstellen der klassifizierten MagnetresonanzMessdaten.

Die Komponenten der Recheneinheit der erfindungsgemäßen Klassifikationseinheit können zum überwiegenden Teil in Form von Softwarekomponenten ausgebildet sein. Grundsätzlich können diese Komponenten aber auch zum Teil, insbesondere wenn es um besonders schnelle Berechnungen geht, in Form von softwareunterstützten Hardwarekomponenten, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden. Selbstverständlich ist es auch denkbar, dass mehrere der genannten Komponenten in Form einer einzelnen Softwarekomponente bzw. softwareunterstützter Hardwarekomponente zusammengefasst realisiert sind.

Das erfindungsgemäße Magnetresonanzgerät umfasst die erfindungsgemäße Klassifikationseinheit.

Die Klassifikationseinheit kann dazu ausgebildet sein, Steuerungssignale an das Magnetresonanzgerät zu senden und/oder Steuerungssignale zu empfangen und/oder zu verarbeiten, um ein erfindungsgemäßes Verfahren auszuführen. Die Klassifikationseinheit kann in das Magnetresonanzgerät integriert sein. Die Klassifikationseinheit kann auch separat von dem Magnetresonanzgerät installiert sein. Die Klassifikationseinheit kann mit dem Magnetresonanzgerät verbunden sein.

Das Erfassen der Magnetresonanz-Messdaten kann eine Aufnahme der Magnetresonanz-Messdaten mittels einer Aufnahmeeinheit des Magnetresonanzgeräts umfassen. Die MagnetresonanzMessdaten können dann an die Klassifikationseinheit zur Weiterverarbeitung übergeben werden. Die Klassifikationseinheit kann die Magnetresonanz-Messdaten dann mittels der Messdatenerfassungseinheit erfassen.

Das erfindungsgemäße Computerprogrammprodukt ist direkt in einen Speicher einer programmierbaren Recheneinheit ladbar und weist Programmcode-Mittel auf, um ein erfindungsgemäßes Verfahren auszuführen, wenn das Computerprogrammprodukt in der Recheneinheit ausgeführt wird. Das Computerprogrammprodukt kann ein Computerprogramm sein oder ein Computerprogramm umfassen. Dadurch kann das erfindungsgemäße Verfahren schnell, identisch wiederholbar und robust ausgeführt werden. Das Computerprogrammprodukt ist so konfiguriert, dass es mittels der Recheneinheit die erfindungsgemäßen Verfahrensschritte ausführen kann. Die Recheneinheit muss dabei jeweils die Voraussetzungen wie beispielsweise einen entsprechenden Arbeitsspeicher, eine entsprechende Grafikkarte oder eine entsprechende Logikeinheit aufweisen, so dass die jeweiligen Verfahrensschritte effizient ausgeführt werden können. Das Computerprogrammprodukt ist beispielsweise auf einem computerlesbaren Medium gespeichert oder auf einem Netzwerk oder Server hinterlegt, von wo es in den Prozessor einer lokalen Recheneinheit geladen werden kann. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in einer Recheneinheit ein erfindungsgemäßes Verfahren ausführen. So kann das Computerprogrammprodukt auch den elektronisch lesbaren Datenträger darstellen. Beispiele für elektronisch lesbare Datenträger sind eine DVD, ein Magnetband, eine Festplatte oder ein USB-Stick, auf welchem elektronisch lesbare Steuerinformationen, insbesondere Software (vgl. oben), gespeichert ist. Wenn diese Steuerinformationen (Software) von dem Datenträger gelesen und in eine Steuerung und/oder Recheneinheit gespeichert werden, können alle erfindungsgemäßen Ausführungsformen der vorab beschriebenen Verfahren durchgeführt werden. So kann die Erfindung auch von dem besagten computerlesbaren Medium und/oder dem besagten elektronisch lesbaren Datenträger ausgehen.

Die Vorteile der erfindungsgemäßen Klassifikationseinheit, des erfindungsgemäßen Magnetresonanzgeräts und des erfindungsgemäßen Computerprogrammprodukts entsprechen im Wesentlichen den Vorteilen der erfindungsgemäßen Verfahren, welche vorab im Detail ausgeführt sind. Hierbei erwähnte Merkmale, Vorteile oder alternative Ausführungsformen sind ebenso auch auf die anderen beanspruchten Gegenstände zu übertragen und umgekehrt. Mit anderen Worten können die gegenständlichen Ansprüche auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module, insbesondere durch Hardware-Module, ausgebildet.

Im Folgenden wird die Erfindung anhand der in den Figuren dargestellten Ausführungsbeispiele näher beschrieben und erläutert. Es zeigen:
Fig. 1 ein erfindungsgemäßes Magnetresonanzgerät mit einer erfindungsgemäßen Klassifikationseinheit in einer schematischen Darstellung,
Fig. 2 ein Ablaufdiagramm der gemeinsamen Schritte einer ersten und zweiten Ausführungsform,
Fig. 3 ein Ablaufdiagramm eines Verfahrens, welches nicht Teil der beanspruchten Erfindung ist,
Fig. 4 ein Ablaufdiagramm einer ersten Ausführungsform eines erfindungsgemäßen Verfahrens und
Fig. 5 ein Ablaufdiagramm einer zweiten Ausführungsform eines erfindungsgemäßen Verfahrens.

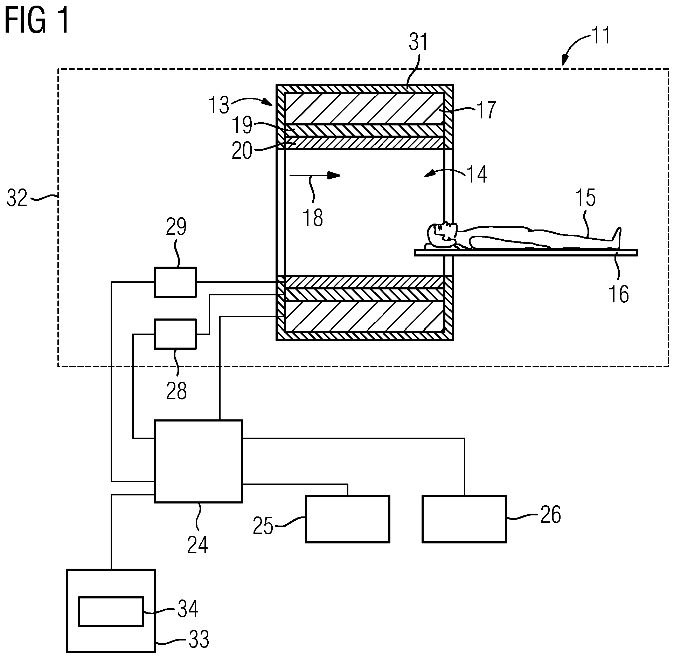

**Fig.** 1 stellt ein erfindungsgemäßes Magnetresonanzgerät 11 mit einer erfindungsgemäßen Klassifikationseinheit 33 schematisch dar.

Das Magnetresonanzgerät 11 umfasst eine von einer Magneteinheit 13 gebildete Detektoreinheit mit einem Hauptmagneten 17 zu einem Erzeugen eines starken und insbesondere konstanten Hauptmagnetfelds 18. Zudem weist das Magnetresonanzgerät 11 einen zylinderförmigen Patientenaufnahmebereich 14 zu einer Aufnahme eines Untersuchungsobjekts 15, im vorliegenden Fall eines Patienten, auf, wobei der Patientenaufnahmebereich 14 in einer Umfangsrichtung von der Magneteinheit 13 zylinderförmig umschlossen ist. Der Patient 15 kann mittels einer Patientenlagerungsvorrichtung 16 des Magnetresonanzgeräts 11 in den Patientenaufnahmebereich 14 geschoben werden. Die Patientenlagerungsvorrichtung 16 weist hierzu einen Liegentisch auf, der bewegbar innerhalb des Magnetresonanzgeräts 11 angeordnet ist. Die Magneteinheit 13 ist mittels einer Gehäuseverkleidung 31 des Magnetresonanzgeräts nach außen hin abgeschirmt.

Die Magneteinheit 13 weist weiterhin eine Gradientenspuleneinheit 19 zu einer Erzeugung von Magnetfeldgradienten auf, die für eine Ortskodierung während einer Bildgebung verwendet werden. Die Gradientenspuleneinheit 19 wird mittels einer Gradientensteuereinheit 28 angesteuert. Des Weiteren weist die Magneteinheit 13 eine Hochfrequenzantenneneinheit 20, welche im gezeigten Fall als fest in das Magnetresonanzgerät 11 integrierte Körperspule ausgebildet ist, und eine Hochfrequenzantennensteuereinheit 29 zu einer Anregung einer Polarisation, die sich in dem von dem Hauptmagneten 17 erzeugten Hauptmagnetfeld 18 einstellt, auf. Die Hochfrequenzantenneneinheit 20 wird von der Hochfrequenzantennensteuereinheit 29 angesteuert und strahlt hochfrequente Magnetresonanz-Sequenzen in einen Untersuchungsraum, der im Wesentlichen von dem Patientenaufnahmebereich 14 gebildet ist, ein. Die Hochfrequenzantenneneinheit 20 ist weiterhin zum Empfang von Magnetresonanz-Signalen, insbesondere aus dem Patienten 15, ausgebildet.

Zu einer Steuerung des Hauptmagneten 17, der Gradientensteuereinheit 28 und der Hochfrequenzantennensteuereinheit 29 weist das Magnetresonanzgerät 11 eine Steuereinheit 24 auf. Die Steuereinheit 24 steuert zentral das Magnetresonanzgerät 11, wie beispielsweise das Durchführen einer vorbestimmten bildgebenden Gradientenechosequenz. Steuerinformationen wie beispielsweise Bildgebungsparameter, sowie rekonstruierte Magnetresonanz-Bilder können auf einer Anzeigeeinheit 25, des Magnetresonanzgeräts 11 für einen Benutzer bereitgestellt werden. Zudem weist das Magnetresonanzgerät 11 eine Eingabeeinheit 26 auf, mittels derer Informationen und/oder Parameter während eines Messvorgangs von einem Benutzer eingegeben werden können. Die Steuereinheit 24 kann die Gradientensteuereinheit 28 und/oder die Hochfrequenzantennensteuereinheit 29 und/oder die Anzeigeeinheit 25 und/oder die Eingabeeinheit 26 umfassen.

Das Magnetresonanzgerät 11 umfasst weiterhin eine Akquisitionseinheit 32. Die Akquisitionseinheit 32 wird im vorliegenden Fall von der Magneteinheit 13 zusammen mit der Hochfrequenzantennensteuereinheit 29 und der Gradientensteuereinheit 28 gebildet.

Das dargestellte Magnetresonanzgerät 11 kann selbstverständlich weitere Komponenten umfassen, die Magnetresonanzgeräte 11 gewöhnlich aufweisen. Eine allgemeine Funktionsweise eines Magnetresonanzgeräts 11 ist zudem dem Fachmann bekannt, so dass auf eine detaillierte Beschreibung der weiteren Komponenten verzichtet wird.

Die dargestellte Klassifikationseinheit 33 umfasst eine Recheneinheit 34. Diese Recheneinheit 34 kann verschiedene Komponenten, wie beispielsweise eine Messdatenerfassungseinheit, eine Ableitungseinheit, einen Klassifikator oder eine Bereitstellungseinheit, umfassen, um ein erfindungsgemäßes Verfahren auszuführen.

Das Magnetresonanzgerät 11 ist somit zusammen mit der Klassifikationseinheit 33 zur Ausführung eines erfindungsgemäßen Verfahrens ausgelegt. Die Recheneinheit 34 der Klassifikationseinheit 33 erfasst dann die Magnetresonanz-Messdaten von der Steuereinheit 24 des Magnetresonanzgeräts 11. Dafür ist die Recheneinheit 34 vorteilhafterweise mit der Steuereinheit 24 des Magnetresonanzgeräts 11 hinsichtlich eines Datenaustauschs verbunden. Die klassifizierten MagnetresonanzMessdaten können dann von der Recheneinheit 34 an die Anzeigeeinheit 25 des Magnetresonanzgeräts 11 für die Anzeige übertragen werden.

Alternativ zur Darstellung kann die Klassifikationseinheit 33 auch alleine zur Ausführung eines erfindungsgemäßen Verfahrens ausgelegt sein. Dazu wird die Recheneinheit 34 typischerweise die Magnetresonanzmessdaten aus einer Datenbank laden und/oder von dem Magnetresonanzgerät 11 abrufen.

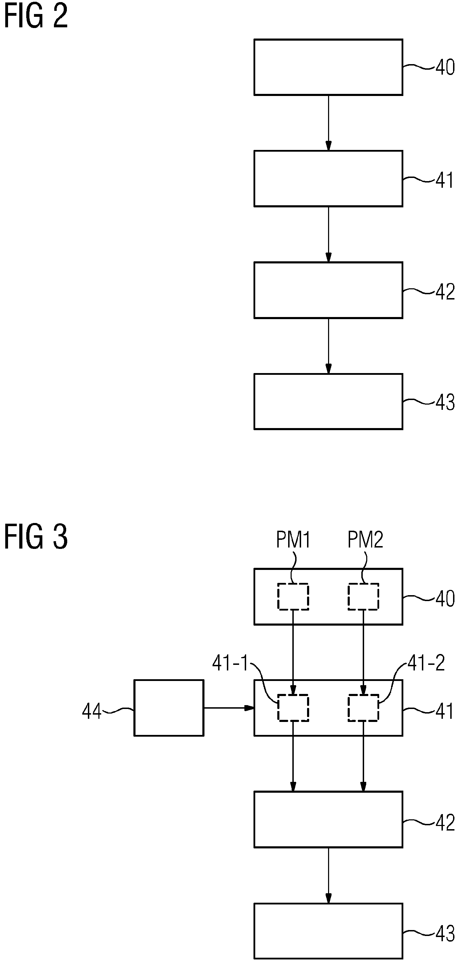

Fig. 2 zeigt ein Ablaufdiagramm der gemeinsamen Schritte der im Folgenden näher beschriebenen erfindungsgemäßen Verfahren zur Klassifikation von mittels einer Magnetresonanz-Fingerprinting Methode von einem Untersuchungsobjekt 15 erfassten Magnetresonanz-Messdaten.

In einem ersten Verfahrensschritt 40 erfolgt ein Erfassen von Magnetresonanz-Messdaten des Untersuchungsobjekts, wobei die Magnetresonanz-Messdaten mehrere mittels einer Magnetresonanz-Fingerprinting Methode erfasste MagnetresonanzSignalverläufe umfassen,

In einem weiteren Verfahrensschritt 41 erfolgt ein Ableiten zumindest eines Texturparameters aus den MagnetresonanzMessdaten.

In einem weiteren Verfahrensschritt 42 erfolgt eine Klassifikation der Magnetresonanz-Messdaten in zumindest eine Gewebeklasse unter Verwendung des zumindest einen Texturparameters. In einem weiteren Verfahrensschritt 43 erfolgt ein Bereitstellen der klassifizierten Magnetresonanz-Messdaten.

Die nachfolgende Beschreibung beschränkt sich im Wesentlichen auf die Unterschiede zu dem Ausführungsbeispiel in Fig. 2, wobei bezüglich gleich bleibender Verfahrensschritte auf die Beschreibung des Ausführungsbeispiels in Fig. 2 verwiesen wird. Im Wesentlichen gleich bleibende Verfahrensschritte sind grundsätzlich mit den gleichen Bezugszeichen beziffert.

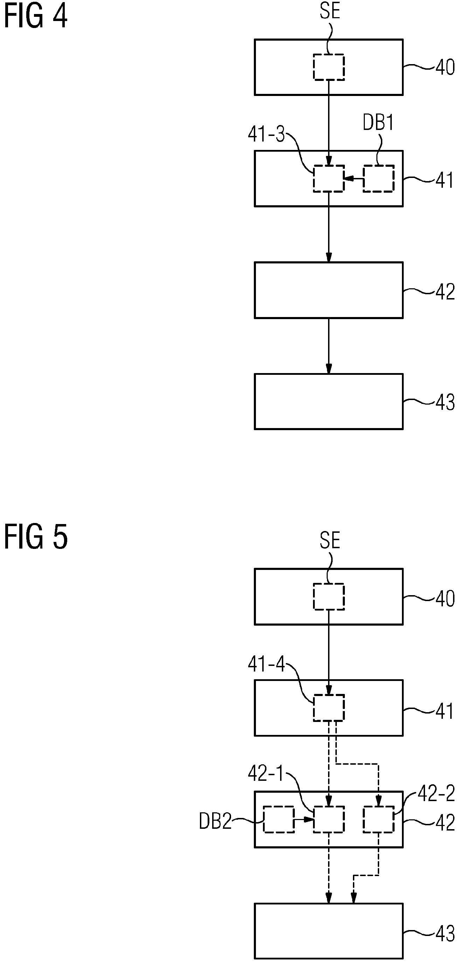

Die in Fig. 4, 5 gezeigten Ausführungsformen des erfindungsgemäßen Verfahrens umfassen im Wesentlichen die Verfahrensschritte 40, 41, 42, 43 gemäß Fig. 2. Zusätzlich umfassen die in Fig. 4, 5 gezeigten Ausführungsformen des erfindungsgemäßen Verfahrens zusätzliche Verfahrensschritte und Unterschritte. Denkbar ist auch ein zu Fig. 4, 5 alternativer Verfahrensablauf, welcher nur einen Teil der in Fig. 4, 5 dargestellten zusätzlichen Verfahrensschritte und/oder Unterschritte aufweist, sofern ein solches Verfahren unter den Gegenstand der unabhängigen Ansprüche fällt. Selbstverständlich kann auch ein zu Fig. 4, 5 alternativer Verfahrensablauf zusätzliche Verfahrensschritte und/oder Unterschritte aufweisen.

**Fig. 3** zeigt ein Ablaufdiagramm eines Verfahrens, welches nicht Teil der Erfindung ist, zur Klassifikation von mittels einer Magnetresonanz-Fingerprinting Methode von einem Untersuchungsobjekt 15 erfassten Magnetresonanz-Messdaten.

Im in Fig. 3 gezeigten Fall umfassen die MagnetresonanzMessdaten mehrere aus den mehreren Magnetresonanz-Signalverläufen rekonstruierte ortsaufgelöste Gewebeparameterkarten PM1, PM2. Exemplarisch liegen dabei eine erste Gewebeparameterkarte PM1 und eine zweite Gewebeparameterkarte PM2. Selbstverständlich können die Magnetresonanz-Messdaten auch mehr Gewebeparameterkarten PM1, PM2 umfassen. Die erste Gewebeparameterkarte PM1 und die zweite Gewebeparameterkarte PM2 quantifizieren dabei unterschiedliche Gewebeparameter. Beispielsweise kann die erste Gewebeparameterkarte PM1 eine ortsaufgelöste Verteilung einer T1-Relaxationszeit darstellen und die zweite Gewebeparameterkarte PM2 kann eine ortsaufgelöste Verteilung einer T2-Relaxationszeit darstellen. Selbstverständlich sind andere Kombinationen von Gewebeparametern denkbar, die in den Gewebeparameterkarten quantifiziert werden.

Im weiteren Verfahrensschritt 41 umfasst nun das Ableiten des zumindest einen Texturparameters ein Ableiten von mehreren Texturparameterkarten aus den mehreren ortsaufgelösten Gewebeparameterkarten in zwei Teilschritten 41-1, 41-2. In einem ersten Teilschritt 41-1 des weiteren Verfahrensschritts 41 wird dabei im in Fig. 3 gezeigten Fall eine erste Texturparameterkarte aus der ersten Gewebeparameterkarte PM1 abgeleitet. In einem zweiten Teilschritt 41-2 des weiteren Verfahrensschritts 41 wird im in Fig. 3 gezeigten Fall eine zweite Texturparameterkarte aus der zweite Gewebeparameterkarte PM1 abgeleitet. Die beiden Texturparameterkarten können somit Informationen über räumliche und/oder zeitliche Strukturen in der jeweils zugehörigen Gewebeparameterkarte PM1, PM2 enthalten. Selbstverständlich können auch mehr Texturparameterkarten aus den Gewebeparameterkarten PM1, PM2 abgeleitet werden. So können zum Beispiel zur ersten Gewebeparameterkarte PM1 im ersten Teilschritt 41-1 zwei oder mehr als zwei verschiedene Texturparameterkarten bestimmt werden, welche unterschiedliche Muster in der ersten Gewebeparameterkarte PM1 charakterisieren. Die zweite Gewebeparameterkarte PM2 kann dann analog zur ersten Gewebeparameterkarte PM1 analysiert werden.

Die Klassifikation der Magnetresonanz-Messdaten im weiteren Verfahrensschritt 42 erfolgt dann unter Verwendung der mehreren Texturparameterkarten, im in Fig. 3 gezeigten Fall unter Verwendung der ersten Texturparameterkarte und der zweiten Texturparameterkarte.

In Fig. 3 ist ein weiterer Verfahrensschritt 44 dargestellt, in dem zumindest eine Längenskala im Ortsraum, auf deren Basis der zumindest eine Texturparameter abgeleitet wird, basierend auf zumindest einem der folgenden Elemente bestimmt wird: eine anatomische Region, von der die MagnetresonanzMessdaten erfasst worden sind, eine klinische Fragestellung für das Erfassen der Magnetresonanz-Messdaten, eine bei dem Untersuchungsobjekt zu erwartende Pathologie, zuvor von dem Untersuchungsobjekt erfasste medizinische Bilddaten. Dieser weitere Verfahrensschritt 44 kann alternativ natürlich auch in den in Fig. 4 oder Fig. 5 beschriebenen Ausführungsformen durchgeführt werden.

Fig. 4 zeigt ein Ablaufdiagramm einer ersten Ausführungsform eines erfindungsgemäßen Verfahrens zur Klassifikation von mittels einer Magnetresonanz-Fingerprinting Methode von einem Untersuchungsobjekt 15 erfassten Magnetresonanz-Messdaten.

Im in Fig. 4 gezeigten Fall werden die im weiteren Verfahrensschritt 40 erfassten mehreren Magnetresonanz-Signalverläufe SE direkt im weiteren Verfahrensschritt 41 weiterverarbeitet.

Dafür umfasst das Ableiten des zumindest einen Texturparameters im weiteren Verfahrensschritt 41 eine Rekonstruktion von zumindest einer Texturparameterkarte in einem Teilschritt 41-3 des weiteren Verfahrensschritts 41, wobei die mehreren Magnetresonanz-Signalverläufe SE in die Rekonstruktion der zumindest einen Texturparameterkarte direkt eingehen. Das in Teilschritt 41-3 für die Rekonstruktion der zumindest einen Texturparameterkarte verwendete Magnetresonanz-Fingerprinting Modell DB1 berücksichtigt dabei einen räumlichen Zusammenhang der meheren Magnetresonanz-Signalverläufe.

Die Klassifikation der Magnetresonanz-Messdaten im weiteren Verfahrensschritt 42 erfolgt dann unter Verwendung der zumindest einen Texturparameterkarte.

**Fig. 5** zeigt ein Ablaufdiagramm einer zweiten Ausführungsform eines erfindungsgemäßen Verfahrens zur Klassifikation von mittels einer Magnetresonanz-Fingerprinting Methode von einem Untersuchungsobjekt 15 erfassten Magnetresonanz-Messdaten.

Im in Fig. 5 gezeigten Fall wird der zumindest eine Texturparameter in einem Teilschritt 41-4 des weiteren Verfahrensschritts 41 zeitaufgelöst über eine Zeitspanne des Erfassens der mehreren Magnetresonanz-Signalverläufe SE abgeleitet.

Die Klassifikation der Magnetresonanz-Messdaten im weiteren Verfahrensschritt 42 kann dann mittels zweier Alternativen erfolgen.

Gemäß der ersten Alternative erfolgt die Klassifikation der Magnetresonanz-Messdaten aus dem zumindest einem zeitaufgelöstem Texturparameter unter Verwendung einer Datenbank DB2 in einem ersten Teilschritt 42-1 des weiteren Verfahrensschritts 42, wobei die Datenbank DB2 Informationen über eine zeitliche Evolution des zumindest einen Texturparameters über die Zeitspanne des Erfassens der mehreren MagnetresonanzSignalverläufe SE in Abhängigkeit von der zumindest einen Gewebeklasse umfasst.

Gemäß der zweiten Alternative erfolgt die Klassifikation der Magnetresonanz-Messdaten aus dem zumindest einem zeitaufgelöstem Texturparameter unter Verwendung zumindest einer Filteroperation in einem zweiten Teilschritt 42-2 des weiteren Verfahrensschritts 42. Die Filteroperation kann auch in den anderen Verfahren gemäß Fig. 3 oder Fig. 4 als Alternative zum direkten Vergleich der modellierten Magnetresonanz-Signalverläufe mit den Datenbank-Signalverläufen eingesetzt werden.

Die in Fig. 4, 5 dargestellten Verfahrensschritte des erfindungsgemäßen Verfahrens werden von der Recheneinheit ausgeführt. Hierzu umfasst die Recheneinheit erforderliche Software und/oder Computerprogramme, die in einer Speichereinheit der Recheneinheit gespeichert sind. Die Software und/oder Computerprogramme umfassen Programmmittel, die dazu ausgelegt sind, das erfindungsgemäße Verfahren auszuführen, wenn das Computerprogramm und/oder die Software in der Recheneinheit mittels einer Prozessoreinheit der Recheneinheit ausgeführt wird.

Obwohl die Erfindung im Detail durch die bevorzugten Ausführungsbeispiele näher illustriert und beschrieben wurde, so ist die Erfindung dennoch nicht durch die offenbarten Beispiele eingeschränkt und andere Variationen können vom Fachmann hieraus abgeleitet werden, ohne den Schutzumfang der Erfindung zu verlassen, welcher durch die Ansprüche gegeben ist.

## Patentansprüche

1. Verfahren zur Klassifikation von mittels einer Magnetresonanz-Fingerprinting Methode von einem Untersuchungsobjekt erfassten Magnetresonanz-Messdaten, umfassend folgende Verfahrensschritte:
- Erfassen von Magnetresonanz-Messdaten des Untersuchungsobjekts, wobei die Magnetresonanz-Messdaten mehrere mittels einer Magnetresonanz-Fingerprinting Methode erfasste Magnetresonanz-Signalverläufe von verschiedenen Voxels umfassen,
- Ableiten zumindest eines Texturparameters aus den Magnetresonanz-Messdaten,
- Klassifikation der Magnetresonanz-Messdaten in zumindest eine Gewebeklasse unter Verwendung des zumindest einen Texturparameters, und
- Bereitstellen der klassifizierten Magnetresonanz-Messdaten, wobei
- das Ableiten des zumindest einen Texturparameters eine Rekonstruktion von zumindest einer Texturparameterkarte umfasst, wobei ein für die Rekonstruktion der zumindest einen Texturparameterkarte verwendetes Magnetresonanz-Fingerprinting Modell einen räumlichen Zusammenhang der mehreren Magnetresonanz-Signalverläufe berücksichtigt, und
- die Klassifikation der Magnetresonanz-Messdaten unter Verwendung der zumindest einen Texturparameterkarte erfolgt.

2. Verfahren zur Klassifikation von mittels einer Magnetresonanz-Fingerprinting Methode von einem Untersuchungsobjekt erfassten Magnetresonanz-Messdaten, umfassend folgende Verfahrensschritte:
- Erfassen von Magnetresonanz-Messdaten des Untersuchungsobjekts, wobei die Magnetresonanz-Messdaten mehrere mittels einer Magnetresonanz-Fingerprinting Methode erfasste Magnetresonanz-Signalverläufe von verschiedenen Voxels umfassen,
- Ableiten zumindest eines Texturparameters aus den Magnetresonanz-Messdaten,
- Klassifikation der Magnetresonanz-Messdaten in zumindest eine Gewebeklasse unter Verwendung des zumindest einen Texturparameters und
- Bereitstellen der klassifizierten Magnetresonanz-Messdaten, wobei der zumindest eine Texturparameter zeitaufgelöst über eine Zeitspanne des Erfassens der mehreren MagnetresonanzSignalverläufe abgeleitet wird.

3. Verfahren nach Anspruch 2, wobei die Klassifikation der Magnetresonanz-Messdaten aus dem zumindest einem zeitaufgelöstem Texturparameter unter Verwendung einer Datenbank erfolgt, wobei die Datenbank Informationen über eine zeitliche Evolution des zumindest einen Texturparameters über die Zeitspanne des Erfassens der mehreren MagnetresonanzSignalverläufe in Abhängigkeit von der zumindest einen Gewebeklasse umfasst.

4. Verfahren nach Anspruch 2, wobei die Klassifikation der Magnetresonanz-Messdaten aus dem zumindest einem zeitaufgelöstem Texturparameter unter Verwendung zumindest einer Filteroperation erfolgt.

5. Verfahren nach einem der vorhergehenden Ansprüche, wobei zumindest eine Längenskala im Ortsraum, auf deren Basis der zumindest eine Texturparameter abgeleitet wird, basierend auf zumindest einem der folgenden Elemente bestimmt wird: eine anatomische Region, von der die Magnetresonanz-Messdaten erfasst worden sind, eine klinische Fragestellung für das Erfassen der Magnetresonanz-Messdaten, eine bei dem Untersuchungsobjekt zu erwartende Pathologie, zuvor von dem Untersuchungsobjekt erfasste medizinische Bilddaten.

6. Klassifikationseinheit, umfassend eine Recheneinheit, wobei die Klassifikationseinheit zum Ausführen eines Verfahrens nach einem der vorhergehenden Ansprüche ausgebildet ist.

7. Magnetresonanzgerät mit einer Klassifikationseinheit nach Anspruch 6.

8. Computerprogrammprodukt, welches direkt in einen Speicher einer programmierbaren Recheneinheit ladbar ist, mit einem Programmcode, welcher bei der Ausführung des Programmcodes durch die Recheneinheit diese veranlasst, ein Verfahren nach einem der Ansprüche 1-5 auszuführen.

## Claims

1. Method for classifying magnetic resonance measurement data acquired from an object under examination by means of a magnetic resonance fingerprinting method comprising the following method steps:
- acquiring magnetic resonance measurement data of the object under examination, wherein the magnetic resonance measurement data comprises a plurality of magnetic resonance signal profiles of different voxels acquired by means of a magnetic resonance fingerprinting method,
- deriving at least one texture parameter from the magnetic resonance measurement data,
- classifying the magnetic resonance measurement data into at least one tissue class using the at least one texture parameter and
- providing the classified magnetic resonance measurement data,
wherein
- deriving the at least one texture parameter involves reconstructing at least one texture parameter map, wherein the magnetic resonance fingerprinting model used for reconstructing the at least one texture parameter map takes account of a spatial context of the plurality of magnetic resonance signal profiles, and
- the magnetic resonance measurement data is classified using the at least one texture parameter map.

2. Method for classifying magnetic resonance measurement data acquired from an object under examination by means of a magnetic resonance fingerprinting method comprising the following method steps:
- acquiring magnetic resonance measurement data of the object under examination, wherein the magnetic resonance measurement data comprises a plurality of magnetic resonance signal profiles of different voxels acquired by means of a magnetic resonance fingerprinting method,
- deriving at least one texture parameter from the magnetic resonance measurement data,
- classifying the magnetic resonance measurement data into at least one tissue class using the at least one texture parameter and
- providing the classified magnetic resonance measurement data,
wherein
the at least one texture parameter is derived in a time-resolved manner over a time interval for acquisition of the plurality of magnetic resonance signal profiles.

3. Method according to claim 2, wherein the magnetic resonance measurement data from the at least one time-resolved texture parameter is classified using a database, wherein the database comprises information about a temporal evolution of the at least one texture parameter over the time interval for acquisition of the plurality of magnetic resonance signal profiles as a function of the at least one tissue class.

4. Method according to claim 2, wherein the magnetic resonance measurement data from the at least one time-resolved texture parameter is classified using at least one filter operation.

5. Method according to one of the preceding claims, wherein, in the spatial domain used as the basis for deriving the at least one texture parameter, at least one length scale is determined on the basis of at least one of the following elements: an anatomical region from which the magnetic resonance measurement data has been acquired, a clinical problem underlying the acquisition of the magnetic resonance measurement data, a disease process expected in the object under examination or image data previously acquired from the object under examination.

6. Classifying unit comprising a computing unit, wherein the classifying unit is configured to carry out a method according to one of the preceding claims.

7. Magnetic resonance device with a classifying unit according to claim 6.

8. Computer program product which is directly loadable into a storage device of a programmable computing unit, with a program code which, when the program code is executed by the computing unit, prompts said computing unit to carry out a method according to one of claims 1-5.

## Revendications

1. Procédé de classement de données de mesure de résonance magnétique détectées d'un objet à examiner au moyen d'une méthode fingerprinting de résonance magnétique, comprenant les stades de procédé suivants :
- la détection de données de mesure de résonance magnétique de l'objet à examiner, les données de mesure de résonance magnétique comprenant plusieurs courbes de signal de résonance magnétique de divers voxels détectées au moyen d'une méthode fingerprinting de résonance magnétique,
- la déduction d'au moins un paramètre de texture à partir des données de mesure de résonance magnétique,
- le classement des données de mesure de résonance magnétique en au moins une classe de tissu en utilisant le au moins un paramètre de texture, et
- la mise à disposition des données de mesure de résonance magnétique classées,
dans lequel
- la déduction du au moins un paramètre de texture comprend une reconstruction d'au moins une carte de paramètre de texture, dans lequel un modèle fingerprinting de résonance magnétique, utilisé pour la reconstruction de la au moins une carte de paramètre de texture, prend en compte une relation spatiale entre les plusieurs courbes de signal de résonance magnétique, et
- le classement des données de mesure de résonance magnétique s'effectue en utilisant la au moins une carte de paramètre de texture.

2. Procédé de classement de données de mesure de résonance magnétique détectées d'un objet à examiner au moyen d'une méthode fingerprinting de résonance magnétique, comprenant les stades de procédé suivants :
- la détection de données de mesure de résonance magnétique de l'objet à examiner, les données de mesure de résonance magnétique comprenant plusieurs courbes de signal de résonance magnétique de divers voxels détectées au moyen d'une méthode fingerprinting de résonance magnétique,
- la déduction d'au moins un paramètre de texture à partir des données de mesure de résonance magnétique,
- le classement des données de mesure de résonance magnétique en au moins une classe de tissu en utilisant le au moins un paramètre de texture, et
- la mise à disposition des données de mesure de résonance magnétique classées,
dans lequel
on déduit le au moins un paramètre de texture en résolution dans le temps sur un laps de temps de la détection des plusieurs courbes de signaux de résonance magnétique.

3. Procédé suivant la revendication 2, dans lequel le classement des données de mesure de résonance magnétique s'effectue à partir du au moins un paramètre de texture en résolution dans le temps en utilisant une base de données, la base de données contenant des informations sur une évolution dans le temps du au moins un paramètre de texture sur le laps de temps de la détection des plusieurs courbes de signal de résonance magnétique en fonction de la au moins une classe de tissu.

4. Procédé suivant la revendication 2, dans lequel le classement des données de mesure de résonance magnétique s'effectue à partir du au moins un paramètre de texture en résolution dans le temps en utilisant au moins une opération de filtrage.

5. Procédé suivant l'une des revendications précédentes, dans lequel on détermine au moins une échelle de longueur dans l'espace de position, sur la base de laquelle on déduit le au moins un paramètre de texture, en se basant au moins sur l'un des éléments suivants : une région anatomique, dont les données de mesure de résonance magnétique ont été détectées, une question clinique pour la détection des données de mesure de résonance magnétique, une pathologie à escompter de l'objet en examen, avant des données d'image médicale détectées de l'objet en examen.

6. Unité de classement, comprenant une unité informatique, l'unité de classement étant constituée pour exécuter un procédé suivant l'une des revendications précédentes.

7. Appareil de résonance magnétique, ayant une unité de classement suivant la revendication 6.

8. Produit de programme d'ordinateur, qui peut être chargé directement dans une mémoire d'une unité informatique programmable, comprenant un code de programme, qui, lors de la réalisation du code de programme par l'unité informatique, fait que celle-ci exécute un procédé suivant l'une des revendications 1 à 5.
